# EUROPÄISCHE PATENTSCHRIFT

(11) **EP 0 714 441 B1**
(45) Veröffentlichungstag und Bekanntmachung des Hinweises auf die Patenterteilung: **04.12.2002**
(21) Anmeldenummer: 94923586.5
(22) Anmeldetag: 24.08.1994
(51) Int. Cl.: C12N 15/31, C07K 14/37, C12N 1/21, A61K 39/35, G01N 33/569, C12N 15/62

(54) **REKOMBINANTE CHLADOSPORIUM HERBARUM ALLERGENE**
RECOMBINANT CLADOSPORIUM HERBARUM ALLERGENS
ALLERGENES RECOMBINES DE CLADOSPORIUM HERBARUM

(30) Priorität: 27.08.1993 AT 172593
(43) Veröffentlichungstag der Anmeldung: 05.06.1996
(73) Patentinhaber: Biomay Produktions- und Handelsgesellschaft mbH, 4020 Linz (AT)
(72) Erfinder: ACHATZ, Gernot, A-5020 Salzburg (AT); OBERKOFLER, Hannes, A-5732 Mühlbach (AT); SIMON, Birgit, A-8700 Leoben (AT); UNGER, Andrea, A-5201 Seekirchen (AT); LECHENAUER, Erich, A-5400 Hallein (AT); HIRSCHWEHR, Reinhold, A-1160 Wien (AT); EBNER, Christoph, A-1040 Wien (AT); KRAFT, Dietric, A-1170 Wien (AT); PRILLINGER, Hans-Jörg, A-3711 Ebersbrunn (AT); BREITENBACH, Michael, A-5020 Salzburg (AT)
(74) Vertreter: Casati, Wilhelm, Dipl.-Ing.
(86) Internationale Anmeldenummer: AT9400120
(87) Internationale Veröffentlichungsnummer: WO95006121

(56) Entgegenhaltungen:
- CLINICAL AND INVESTIGATIVE MEDICINE, Bd. 16,Nr. 4sup, August 1993 Seite B6 L.ZHANG ET AL 'Molecular cloning and characterization of allergens of Cladosporium herbarum' & Annual meeting of the Canadian Society for Clinical Investigation
- THE JOURNAL OF ALLERGY AND CLINICAL IMMUNOLOGY, Bd. 83,Nr. 1, 1989 Seite 292 M. BURTON ET AL 'Characterization of allergens from six isolates of Cladosporium herbarum'
- THE JOURNAL OF ALLERGY AND CLINICAL IMMUNOLOGY, Bd. 91,Nr. 1, Januar 1993 Seite 273 L. ZHANG ET AL 'Purification and characterization of a high molecular weight antigen from Cladosporium herbarum'
- INT. ARCHS. ALLERGY APPL. IMMUN., Bd. 78, 1985 Seiten 249-255, M. SWÄRD-NORDMO ET AL 'Purification and partial characterization of the allergen Ag-54 from Cladosporium herbarum'
- PROCEEDINGS OF THE NATIONAL ACADEMY OF SCIENCES OF USA, Bd. 80, März 1983 WASHINGTON US, Seiten 1194-1198, R. YOUNG AND R. DAVIS 'Efficient isolation of genes by using antibody probes'
- THE JOURNAL OF ALLERGY AND CLINICAL IMMUNOLOGY, Bd. 89,Nr. 1, Januar 1992 Seite 241 H. SANCHEZ ET AL 'cDNA sequence of an Alternaria allergen'
- EMBO JOURNAL, Bd. 8, 1989 Seiten 1935-1938, H. BREITENEDER 'The gene coding for the major birch pollen allergen BetvI, is highly homologous to a pea disease resistance response gene'
- JOURNAL OF BIOLOGICAL CHEMISTRY, Bd. 266,Nr. 2, 15.Januar 1991 MD US, Seiten 1204-1210, A. SILVANOVICH ET AL 'Nucleotide sequence and analysis of three cDNAs coding for Poa p IX isoallergens of Kentucky Bluegrass pollen'
- JOURNAL OF IMMUNOLOGY, Bd. 138,Nr. 7, 1.April 1987 Seiten 2213-2229, H. MARGALIT ET AL 'Prediction of immunodominant helper T cell antigenic sites from the primary sequence' in der Anmeldung erwähnt
- THE JOURNAL OF ALLERGY AND CLINICAL IMMUNOLOGY, Bd. 93,Nr. 1, Januar 1994 Seite 207 L. ZHANG ET AL 'A novel allergen of Cladosporium herbarum identified as a ribosomal P2 protein'

## Beschreibung

Die Erfindung bezieht sich auf rekombinante DNA-Moleküle, die für das Allergen Clah47 kodieren.

Die Allergene des extramuralen Schimmelpilzes Cladosporium herbarum, so-wie auf die Molekülfragmente (B-und T-Zell stimulierende Peptide) führen im Rahmen einer Immunantwort zu einem Überschießen der IgE Antikörperproduktion bei Pilzallergikem. Rekombinante Allergene bzw. immunogen wirkende Teilpeptide können sowohl in vitro als auch in vivo zu einer verbesserten Diagnostik von Schimmelpilzallergien aber auch zur Induktion einer Immuntoleranz bzw. Anergie von allergen-spezifischen T-Zellen herangezogen werden.

Das Immunsystem der Vertebraten entwickelte sich in der Evolution als wirksame Waffe gegen Angriffe auf das Individuum von außen, aber auch von innen. Unter "normalen" Bedingungen kann das Immunsystem zwischen "Selbst" und "Nicht-Selbst" unterscheiden. Wie man aber heute von vielen Regulationskaskaden weiß, laufen solche Regulationsmechanismen nicht immer fehlerlos ab, was im Falle der Immunologie einem Angriff auf eigenes Gewebe gleichkommt. Man kennt heute mehrere prinzipielle Situationen, in denen der Körper Opfer seines eigenen Immunsystems wird. Eine dieser unerwünschten immunologischen Antworten kann durch Umweltantigene ausgelöst werden. Die Reaktion, die durch diese Fehlsteuerung verursacht wird, nennt man Allergie oder Hypersensibilitäts-Reaktion. Gell und Coombs (1975) definierten 4 Hypersensibilitätstypen (Typ I,II,III und IV). Allergene wie Pilzsporen gehören zu den "Typ I" oder "Anaphylaxie-Hypersensibilitäts" auslösenden Antigenen.

Das typische Bild einer Typ I Hypersensibilität besteht darin, daß ein einmali-ger Kontakt mit einem bestimmten Antigen (z.B. mit Pilzsporen) keinen nennenswerten Effekt auf das Individuum zeigt. Kommt es jedoch nach einigen Wochen ein zweites Mal zu einem Allergenkontakt, dann reagiert der nun sensibilisierte Organismus mit Symptomen einer allgemeinen Anaphylaxie. Eine Kontraktion der glatten Muskulatur sowie eine Dilatation der Kapillaren ist die Folge. Dies beruht darauf, daß der primäre Kontakt mit dem allergenen Proteine in einer ungewöhnlichen humoralen Immunantwort resultiert. Neben einer in diesem Fall erwünschten IgG-Antwort, antwortet der Allergiepatient mit einer massiven IgE-Produktion. Diese primär gebildeten IgEs, binden mit ihren Fc-Teilen an spezifische hochaffine Fc-Epsilon-Rezeptoren, deren Hauptlokalisation an der Außenseite von Mastzellen und Basophilen zu finden ist. Bei einem Zweitkontakt mit dem Allergen kommt es zwischen den gebundenen IgE-Molekülen durch das Allergen zu einer Quervernetzung, was schließlich eine Degranulation der Mastzellen und Basophilen und somit die Freisetzung von Mediatorstoffen wie Histamin und Arachidonsäuremetaboliten etc. zur Folge hat.

Die wichtigsten Umweltallergene sind Proteine mit einem Molekulargewichte zwischen 10 und 50kD. Die wichtigste Quelle dieser allergenen Proteine, die eine TypI-Allergie hervorrufen, stellen Inhalationsallergene wie Pilzsporen, Pollen, Kot von Hausstaubmilben etc. dar (Review Bold et al. 1973). Die für eine Pilzallergie relevanten Pilze gehören einer Gruppe von eukaryontischen, filamentös wachsenden, Sporen bildenden Pilzen an. Da die Sporen die Verbreitungsform des Pilzes darstellen (die Sporen können durch den Wind leicht vertragen werden), ist anzunehmen, daß ihnen eine entscheidende Rolle bei der Allergieauslösung zukommt.

Man weiß heute, daß 20% der Atopiker durch Pilzsporen sensibilisiert wurden (Lazey 1981). Solche Patienten zeigen, wenn der obere Atmungstrakt mit Pilzsporen in Kontakt gekommen ist, eine typische TypI-Allergie mit Symptomen wie Heuschnupfen und Asthma. Wenn Pilzsporen in einer solchen TypI-Antwort involviert sind, beträgt die Größe der Sporen mehr als 5µm. Die Größenselektion begünstigt Pilze wie Cladosporium herbarum und Alternaria alternata als Allergieauslöser.

Cladosporium nerbarum (bzw. die Sporen von Cladosporium herbarum) ist der am häufigsten vorkommende Pilz in der Luft (Gravesen 1979). Die sehr trockenen Sporen von Cladosporium herbarum können durch den Wind relativ leicht vertragen werden. In belasteten Zeiten ist es nicht selten, daß man an die 3 5000 Konidien pro m³ Luft findet. Durch die leichte Verschleppung der Sporen ist an solchen Spitzentagen auch in geschlossenen Räumen eine erhöhte Sporenbelastung meßbar. Die Hauptbelastungszeit liegt zwischen Frühling und Frühherbst. Diese hohe Konidienzahlen lassen sich damit erklären, daß Cladosporium herbarum wegen seiner "genügsamen" Lebensweise nahezu überall zu finden ist. Bevorzugte Lebensräume sind jedoch absterbende Pflanzen, verschiedene Bodentypen, aber auch diverseste Nahrungsmitteln. Nicht gereinigte Kühlschränke, Fensterrahmen, Strohdächer und verschiedene Textilien gehören zu den weiteren Stand- bzw. Lebensorten dieses Pilzes.

Aus diesen Gründen (ein Kontakt mit Cladosporium herbarum Sporen kann praktisch niemals gänzlich ausgeschlossen werden) ist es nicht verwunderlich, daß Cladosporium herbarum zum Gegenstand intensiver allergologischer Forschung geworden ist. So reagieren zB. in Finnland 8% der asthmatischen Kinder positiv auf Cladosporium (Foucard et al. 1984).

Die Beschreibung der allergenen Proteine von Cladosporium herbarum erfolgt mittels zum Teil aufwendiger molekularbiologischen Techniken. Die vermutete Zahl von Cladosporium herbarum Allergenen liegt bei ca. 60 (Aukrust 1979, 1980). Das in der Literatur beschriebene Hauptallergen ClahI 1 wurde aus Rohextrakten aufgereinigt. Das Molekulargewicht liegt bei etwa 13kD. Klonierungen von diversen Cladosporium herbarum Allergenen sind noch nicht vorgenommen worden. Der Vorteil von gentechnologisch hergestellten allergenen Proteinen bzw. deren Teilpeptiden (Voraussetzung dafür ist jedoch eine immunologisch vergleichbare Reaktivität - konnte bei Betula verucosa (Ferreira et al. 1993) und anderen Allegenen schon gezeigt werden) liegt:
a) In der Verbesserung der Testsysteme wie RIA (Radioimmunassay), IRMA (Immunradiometrische Assay), ELISA (Enzyme-linked immunosorbent Assay), LIA (luminescense immunoassay), Immunblots, Histamine-release-assay, T-Zell Proliferationsassay und viele mehr.
b) In der Verbesserung der Hyposensibilisierungstherapie: Diese Therapie besteht in der Zufuhr von Allergenextrakten in Form von Injektionen oder peroraler Applikation in wässriger Form als Tropfen in steigender Dosierung, bis eine Erhaltungsdosis über mehrere Jahre erreicht ist. Resultat dieser Therapie ist das Erreichen einer Toleranz gegenüber den eingesetzten Allergenen, was sich in einer Abnahme der Krankheitssymptome äußert (Birkner et al. 1990). Das Problem bei dieser Art der Behandlung liegt in der Vielzahl der dadurch auftretenden Nebenwirkungen. Bei der Hyposensibilisierungstherapie sind Fälle von anaphylaktischem Schock während der Behandlung aufgetreten. Das Problem hierbei liegt in der schweren Standardisierbarkeit der Pilzprotein-Isolate. Bei einem Einsatz von von Allergenen abgeleiteten aber nicht anaphylaktisch wirkenden Peptiden könnten risikolos höhere Dosen verabreicht werden, wodurch eine wesentliche Verbesserung der Hyposensibilisierung erreicht werden kann.
c) Mit diesen Untersuchungen können aber auch spezifische T- und B-Zell-Epitope definiert werden. Solche Peptide besitzen die Fähigkeit zB. T-Lymphozyten zu stimulieren und zur Proliferation anzuregen, aber die Zellen (bei genau definierter Dosis) auch in einen Zustand der Toleranz bzw. Nicht-Reaktivität (Anergie) zu ver- setzen (Rothbard et al. 1991).

Erfindungsgemäß wird ein rekombinantes DNA Molekül der eingangs genannten Art geschaffen, das eine Nucleinsäuresequenz aufweist, die mit der Sequenz 1 übereinstimmt. Die DNA Moleküle können auch Nucleinsäuresequenzen aufweisen, die durch Degeneration von der vorgenannten Sequenz ableitbar sind.

Weitere Merkmale gehen aus den nachstehenden Darlegungen hervor.

### Beispiele:

### a) Beschreibung der allergenen Proteine von Cladosporium herbarum mittels Westem-Blotting

Für die Klonierung der vorliegenden Allergene von Cladosporium herbarum standen Sera von 142 Atopikern zur Verfügung. Um die Reaktivität der Patienten mit Pilzproteinextrakt zu testen, wurde Cladosporium herbarum (Sammlung Prof. Windisch [Berlin] Nummer: 28-0202) in Flüssigmedium (2% Glukose, 2% Pepton, 1% Hefeextrakt) gezüchtet und anschließend lyophilisiert. Aus diesem Material wur- den sodann die allergenen Proteine ausgewaschen und mittels Lyophilisator auf- konzentriert. Die Auftrennung erfolgte auf einem denaturierenden Polyacrylamidgel, das anschließend geblottet, mit Patientenserum inkubiert und mit ¹²⁵I-markiertem anti human IgE detektiert wurde. In Prozentzahlen ausgedrückt reagierten die Patienten auf das allergene Protein Clah47 zu 53%.

Wurde Protein aus gekauftem Pilzmaterial der Firma Allergon (Schweden) isoliert und für den Immunblot verwendet, konnte nahezu dasselbe Bandenmuster detektiert werden. Somit kann Clah47 in Bezug auf das uns zur Verfügung stehende Patientenspektrum als Hauptallergen eingestuft werden.

Die angeschlossenen zwei Figuren zeigen einen Überblick über das zur Klonierung der beschriebenen Allergene zur Verfügung gestandene Patientenspektrum. Das erste der beiden Bilder zeigt ein 13,5 %iges Acrylamidgel. Die Patienten mit der Nummer 19 und 35 (es handelt sich hier auch um die Patienten, die für das spätere Screenen verwendet wurden) zeigen Banden in der Größenordnung 53kD, 46kD und 22kD. Im zweiten Bild, es handelt sich hier um ein 17,5% Polyacrylamidgel, wird auch die kleine Molekulargewichtsbande (11kD) bei Patient 35 sichtbar.

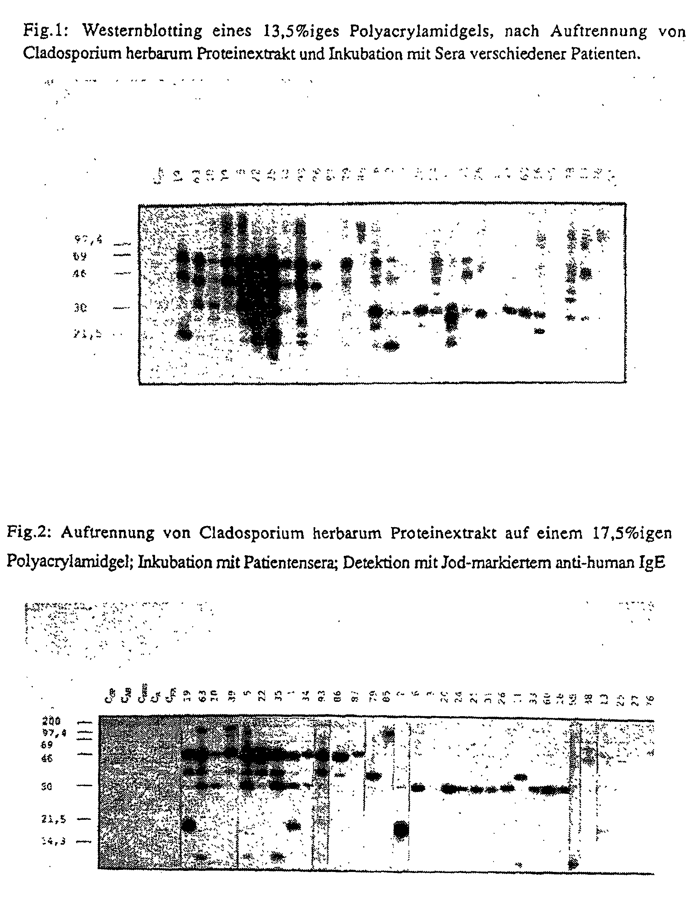

Fig. 1 zeigt ein Westernblotting eines 13,5%iges Polyacrylamidgels nach Auftrennung von Cladosporium herbarum Proteinextrakt und Inkubation mit Sera verschiedener Patienten.

Fig.2 zeigt eine Auftrennung von Cladosporium herbarum Proteinextrakt auf einem 17,5%igen Polyacrylamidgel; Inkubation mit Patientensera; Detektion mit Jod-markiertem anti-human IgE.

### b) Konstruktion der cDNA Expressionsbank

Gesamt RNA wurde nach der sauren Guanidium-Phenol-Extraktionsmethode aus selbst gezüchtetem Pilzmaterial gewonnen. Poly(A)plus mRNA-Anreicherung erfolgte mit Oligo(dT) Cellulose der Firma Böhringer. Die cDNA Synthese (1. und 2. Strang) wurde wie im Manual des Lambda ZAP-Systems der Firma Stratagene beschrieben durchgeführt. Die cDNA wurde anschließend (3'- seitig) mit EcoRI und (5'-seitig) mit Xbal Linkern versehen, in vorverdaute Lambda-ZAP-Arme ligiert und verpackt. Der Titer der Primärbank betrug 1000000 Klone.

### c) Screening der cDNA Genbank mit Patientensera, in vivo Excision, Sequenzierung

Das Screenen der Expressionsbank erfolgte mittels Inkubation der "gelifteten" Phagenplaques mit einem Seragemisch aus 2 Patienten, von denen man durch das Westernblotting wußte, daß sie das Spektrum der detektierten Antigene abdecken. Die Detektion erfolgte wieder mit anti human IgE RAST Antikörper der Firma Pharmacia. Von den nach Sekundär- und Tertiärscreening übriggebliebenen 200 positiven Klonen wurden 30 mit Hilfe eines Helferphagen in vivo exzisiert und zu einem bereits fertig sequenzierbaren Bluescriptvektor religiert (Durchführung wie im Manual des Lambda ZAP-Kits). Restriktionsverdaue der exzisierten Plasmide zeigten (EcoRI-XbaI Doppelverdaue) 4 verschiedene Inserttypen. Diese 4 Klone wurden nach der Sangermethode (Sanger 1977) sequenziert.

### d) Expression von Clah47 als β-Galaktosidasefusionsprotein

Mit Hilfe des vorher beschriebenen IgE-Screenings konnten vier vollständige cDNA-Klone erhalten werden. Die jeweiligen rekombinanten Plasmide wurden in den E.coli Stamm XLl-Blue transformiert und mit IPTG (Isopropyl-β-D-thiogalactopyranosid) induziert. Der E.coli Gesamtproteinextrakt wurde ansschließend elektrophoretisch aufgetrennt und und aufNitrozellulose geblottet. Das Fusionsprotein wurde mittels Serum IgE von Pilzallergikern und einem mit einem ¹²⁵I-markiertem Kaninchen-anti human IgE Antikörper (Pharmacia, Uppsala Schweden) detektiert.

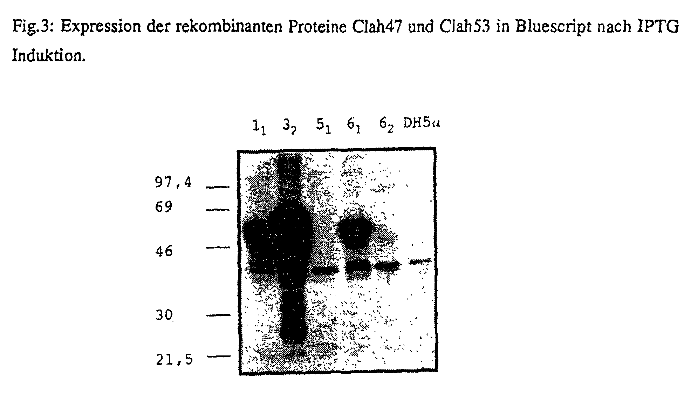

Fig. 3 zeigt das rekombinante β-Galaktosidasefusionsprotein nach Inkubation mit Patientenserum und Detektion mit jodmarkiertem anti-human IgE. Der β-Galaktosidaseanteil des Fusionsproteins beträgt 36 Aminosäuren, was einem Molekulargewicht von 3800 Dalton gleichkommt. Unter Berücksichtigung dieser "Vergrößerung" des allergenen Proteins ist auch dieFig. 3 zu sehen. Spur 1 (Klon 1-1) und 4 (Klon 6-1) zeigen das rekombinante Fusionsprotein Clah47, jetzt um den Fusionsanteil größer. Spur 2 (Klon 3-2) zeigt das rekombinante Clah53 Allergen.

Fig.3 zeigt somit eine Expression der rekombinanten Proteine Clah47 und Clah53 im Vektor BS-SK⁺⁺ nach IPTG Induktion.

### e) Bestimmung von B- und T-Zell Epitopen bei den rekombinanten Allergenen

Die rekombinante Primärsequenz der Allergene bietet die Voraussetzung für die Vorhersage von B- und T-Zellepitopen mittels geeigneter Computerprogramme. Die bestimmten Epitope werden jeweils bei der Beschreibung des rekombinanten Proteins in eigenen Figuren angeführt. Mit diesen Untersuchungen können spezifische T- und B-Zell-Epitope definiert werden die die Fähigkeit besitzen zB T-Lymphozyten zu stimulieren und zur Proliferation anzuregen, aber die Zellen (bei genau definierter Dosis) auch in einen Zustand der Toleranz bzw. Nicht-Reaktivität (Anergie) zu versetzen (Rothbard et al. 1991).

Die Suche nach B-Zellepitopen wurde mit Hilfe des GCG-Programmes (Genetics-Computer-Group) "PROTCALC", das jedoch von der Arbeitsgruppe um Prof Modrow mit wesentlichen Parametern erweitert wurde, durchgeführt. Die Bestimmung beruht auf einer Abwägung der Parameter Hydrophilität (Kyte-Doolittle), Sekundärstruktur (Chou-Fasman), Oberflächenlokalisation (Robson-Garnier) und Flexibilität, wodurch die Antigenität von Teilpeptiden errechnet wird.

Das Prinzip der T-Zellepitop-Voraussage erfolgte im Prinzip nach dem Algorithmus von Margalit et al. (1987). Das Prinzip besteht in der Suche nach amphipathischen Helices laut Primärsequenz des zu bestimmenden Proteins, flankiert von hydrophilen Bereichen. Der berechnete Score muß für relevante T-Zellepitope größer als 10 sein. Bei MHC II (major histo compatibility locus) assoziierten Peptiden kann kein Konsensus, weder der Sequenz noch der Länge des Peptids nach, wie bei HLA-A2 (human leucocyte antigen) assoziierten definiert werden. Bei HLA-A2 assoziierten Peptiden beträgt die Länge des Peptids 10 Aminosäuren, wobei die 2. Aminosäure ein Tyrosin und die letzte Aminosäure ein Leucin darstellt (Rammensee et al. 1993). Die berechneten Epitope werden bei der Beschreibung der einzelnen allergenen Sequenzen getrennt angeführt.

### Molekulare Charakterisierung der klonierten Pilzallergene (Sequenzprotokolle)

Im folgenden Kapitel werden nun die cDNA Sequenzen und die mit ihnen durchgeführten Analysen der Reihe nach angeführt. Die Computerauswertung der nachfolgenden Sequenzen wurden auf einer Ultrix-DEC 5000 Workstation unter Zuhilfename des GCG-Softwarepaketes (=Wisconsin Paket: die Algorithmen dieses Paketes wurden von der Universität Wisconsin entwickelt) durchgeführt.

### Clah47

Die nachfolgende Sequenz 1 zeigt die vollständige cDNA Sequenz des allergenen Proteins Clah47. Die Aminosäuresequenz wurde von der DNA Sequenz hergeleitet. Auch bei diesem Protein sind keine Anzeichen einer Signalsequenz vorhanden. Die Gesamt DNA Sequenz beträgt 1323 Basenpaare, was einer Proteinlänge von 441 Aminosäuren entspricht. Das berechnete Molekulargewicht des rekombinanten Proteins beträgt 47617 Dalton und entspricht somit der detektierten Bande (47kD) im Westernblot. Wie zu Beginn erwähnt, wird das allergene Protein mit dem Molekulargewicht von 47kD von 53% der Patienten erkannt und stellt somit ein wichtiges Hauptallergen dar.

### Sequenz 1: Clah47=Enolase_clado -> 1-phasen Translation 47617 Dalton

(1) ANGABEN ZU SEQ ID NO:1
   (i) SEQUENZKENNZEICHEN:
      (A) LÄNGE: 1323 Basenpaare / 441 Aminosäurereste
      (B) ART: Nukleinsäure / Protein
      (C) STRANGFORM: ds
      (D) TOPOLOGIE: linear
   (ii) ART DES MOLEKÜLS: cDNA zu mRNA / Protein
   (iii) HYPOTHETISCH: nein
   (iv) ANTISENSE: nein
   (v) ART DES FRAGMENTS: Gesamtsequenz
   (vi) URSPRÜNGLICHE HERKUNFT:
      (A) ORGANISMUS: Cladosporium herbarum
      (C) ENTWICKLUNGSSTADIUM: Sporen und vegetative Hyphen

DNA Sequenz 1323 b.p. ATGCCTATCTCC ... ATCAACTTGTAA linear

1 / 1 31 / 11
ATG CCT ATC TCC AAG ATC CAC TCC CGC TAC GTC TAC GAC TCC CGT GGA AAC CCC ACC GTC
met pro ile ser lys ile his ser arg tyr val tyr asp ser arg gly asn pro thr val
61 / 21 91 / 31
GAG GTC GAC ATT GTC ACC GAG ACC GGT CTT CAC CGC GCC ATT GTC CCC TCT GTT GCC TCT
glu val asp ile val thr glu thr gly leu his arg ala ile val pro ser val ala ser
121 / 41 151 / 51
ACC GGC TCC CAC GAG GCT TGC GAG CTC CGT GAT GGT GAC AAG AGC AAG TGG GCT GGC AAG
thr gly ser his glu ala cys glu leu arg asp gly asp lys ser lys trp ala gly lys
181 / 61 211 / 71
GGT GTG ACC AAG GCT GTT GCC AAC GTC AAC GAG ATC ATT GCT CCC GCT CTC ATC AAG GAG
gly val thr lys ala val ala asn val asn glu ile ile ala pro ala leu ile lys glu
241 / 81 271 / 91
AAC CTC GAC GTC AAG GAC CAG GCC GCC GTT GAC GCT TTC CTC AAC AAG CTC GAT GGC ACC
asn leu asp val lys asp gln ala ala val asp ala phe leu asn lys leu asp gly thr

301 / 101 331 / 111
ACC AAC AAG ACC AAG ATT GGT GCC AAC GCC ATC CTC GGT GTC TCC ATG GCT GTG GCC AAG
thr asn lys thr lys ile gly ala asn ala ile leu gly val ser met ala val ala lys
361 / 121 391 / 131
GCT GCC GCC GCC GAG AAG CGT GTC CCT CTC TAC GCC CAC ATC AGC GAC CTT TCC GGC ACC
ala ala ala ala glu lys arg val pro leu tyr ala his ile ser asp leu ser gly thr
421 / 141 451 / 151
AAG AAG CCC TTC GTT CTC CCC GTT CCC TTC ATG AAC GTC GTC AAC GGT GGC TCC CAC GCg
lys lys pro phe val leu pro val pro phe met asn val val asn gly gly ser his ala
481 / 161 511 / 171
GGT GGC CGT CTT GCC TTC CAG GAG TTC ATG ATC GTT CCC AGC Ggg GCT CCC TCC TTC ACC
gly gly arg leu ala phe gln glu phe met ile val pro ser gly ala pro ser phe thr
541 / 181 571 / 191
GAG GCC ATG CGC CAG GGT GCT GAG GTC TAC CAG AAG CTG AAG TCC CTC ACC AAG AAG AGG
glu ala met arg gln gly ala glu val tyr gln lys leu lys ser leu thr lys lys arg
601 / 201 631 / 211

TAC GGC CAG TCT GCC GGC AAC GTC GGT GAC GAG GGT GGT GTC GCT CCC GAT ATT CAG ACC
tyr gly gln ser ala gly asn val gly asp glu gly gly val ala pro asp ile gln thr
661 / 221 691 / 231
GCT GAG GAG GCT CTT GAC CTC ATC ACC GAC GCC ATT GAG GAG GCC GGC TAC ACT GGC CAG
ala glu glu ala leu asp leu ile thr asp ala ile glu glu ala gly tyr thr gly gln
721 / 241 751 / 251
ATC AAG ATC GCC ATG GAC gtt GCC TCC TCC GAG TTC TAC AAG GCC GAC GAG AAG AAG TAC
ile lys ile ala met asp val ala ser ser glu phe tyr lys ala asp glu lys lys tyr
781 / 261 811 / 271
GAC CTt GAC TTC AAG AAC CCC GAC TCT GAC AAG AGC AAG TGG ATC ACC TAC GAG CAG CTT
asp leu asp phe lys asn pro asp ser asp lys ser lys trp ile thr tyr glu gln leu
841 / 281 871 / 291
GCC GAC CAG TAC AAC GAG CTC GCT GCC AAG TAC CCC ATT GTC TCC ATT GAG GAC CCC TTC
ala asp gln tyr asn glu leu ala ala lys tyr pro ile val ser ile glu asp pro phe
901 / 301 931 / 311
GCT GAG GAT GAC TGG GAG GCC TGG AGC TAC TTC TAC AAG ACC TCT GGC TCT GAC TTC CAG
ala glu asp asp trp glu ala trp ser tyr phe tyr lys thr ser gly ser asp phe gln
961 / 321 991 / 331
ATC GTCGGT GAT GAC CTT ACC GTC ACC AAC CCC GAG TTC ATC AAG AAG GCC ATC GAG ACC
ile val gly asp asp leu thr val thr asn pro glu phe ile lys lys ala ile glu thr
1021 / 341 1051 / 351
AAG GCC TGC AAC GCC CTC CTC CTC AAG GTC AAC CAG ATC GGT ACC ATC ACC GAG GCC ATC
lys ala cys asn ala leu leu leu lys val asn gln ile gly thr ile thr glu ala ile
1081 / 361 1111 / 371
AAC GCC GCC AAG GAC TCC TTC GCC GCT GGC TGG GGT GTC ATG GTC TCt caC CGT TCC GGT
asn ala ala lys asp ser phe ala ala gly trp gly val met val ser his arg ser gly
1141 / 381 1171 / 391
GAG ACC GAG GAC GTC ACC atc gcc gac ATC GTT GTC GGT CTC CGT GCC GGC CAG ATc AAG
glu thr glu asp val thr ile ala asp ile val val gly leu arg ala gly gln ile lys
1201 / 401 1231 / 411
ACC GGT GCC CCT GCC CGT TCC GAG CGT CTC GCC AAG CTC AAC CAG ATC CTC CGT ATC GAG
thr gly ala pro ala arg ser glu arg leu ala lys leu asn gln ile leu arg ile glu
1261 / 421 1291 / 431
GAG GAG CTC GGT GAC AAG AGG CTC TAC GCC GGT GAC AAC TTC cgC ACT GCC ATC AAC TTG
glu glu leu gly asp lys arg leu tyr ala gly asp asn phe arg thr ala ile asn leu
1321 / 441
TAA
OCH

Nachfolgende Sequenzvergleiche (Seq.2) der Aminosäuresequenz mit der SWISSPROT Proteinbank ergaben, daß Clah47 signifikante Homologie zu Enolasen zeigt. Im nachfolgenden "multiple sequence alignment" sieht man die hohen Homologien und Identitäten zwischen Clah47 und den übrigen Enolasen (Mensch, Ratte, Maus, Drosophila, Hefe).

### Sequenz 2:

1                                                    50
pileup.msf{enog_human}  .SIqKIWARE IlDSRGNPTV EVDLyTaKGL FRAAVPSGAS TGIyEALELR
pileup.msf{enog_rat}  .SIqKIWARE IlDSRGNPTV EVDLhTaKGL FRAAVPSGAS TGIyEALELR
pileup.msf{enog_mouse}  .SIeKIWARE IlDSRGNPTV EVDLyTaKGL FRAAVPSGAS TGIyEALELR
pileup.msf{eno_drome}  MtIkaIkARq IYDSRGNPTV EVDLTTElGL FRAAVPSGAS TGVHEALELR
pileup.msf{eno1_yeast}  .aVSKVYARs VYDSRGNPTV EVELTTEKGv FRsiVPSGAS TGVHEALEMR
pileup.msf{eno2_yeast}  .aVSKVYARs VYDSRGNPTV EVELTTEKGv FRsiVPSGAS TGVHEALEMR
pileup.msf{Eno_clado}  MpISKIhsRy VYDSRGNPTV EVDivTEtGL hRAiVPSvAS TGsHEAcELR
Consensus  --------R- --DSRGNPTV EV---T--G- -R--VPS-AS TG--EA-E-R

51                                                    100
pileup.msf{enog_human}  DGDKqrYLGK GVLKAVDHIN stIAPALIss gLsVVEQEkl DNLMLELDGT
pileup.msf{enog_rat}  DGDKqrYLGK GVLKAVDHIN stIAPALIss gLsVVEQEkl DNLMLELDGT
pileup.msf{enog_mouse}  DGDKqrYLGK GVLKAVDHIN srIAPALIss gisVVEQEkl DNLMLELDGT
pileup.msf{eno_drome}  DnDKanYhGK sVLKAVgHVN dtlgPeLIKa NLDVVDQasI DNFMikLDGT
pileup.msf{eno1_yeast}  DGDKskWMGK GVLhAVknVN dvIAPAFVKa NlDVkDQkaV DdFLisLDGT
pileup.msf{eno2_yeast}  DeDKskWMGK GVMnAVnnVN nvIAaAFVKa NLDVkDQkaV DdFLLsLDGT
pileup.msf{Eno_clado}  DGDKskWaGK GVtKAVanVN eiIAPALIKe NLDVkDQaaV DaFLnkLDGT
Consensus  D-DK----GK -V--AV---N ---------- ---V--Q--- D-----LDGT

101 150
pileup.msf{enog_human} ENKSKFGANA ILGVSLAVcK AGAAErE1PL YRHIAqLAGN ..SDLILPVP
pileup.msf{enog_rat} ENKSKFGANA ILGVSLAVcK AGAAEKD1PL YRHIAqLAGN ..SDLILPVP
pileup.msf{enog_mouse} ENKSKFGANA ILGVSLAVcK AGAAErD1PL YRHIAqLAGN ..SDLILPVP
pileup.msf{eno_drome} ENKSKFGANA ILGVSLAVaK AGAAkKgVPL YkHIADLAGN ..kEiILPVP
pileup.msf{eno1_yeast} aNKSKLGANA ILGVSLAasr AaAAEKnVPL YkH1ADLsks KTSpYVLPVP
pileup.msf{eno2_yeast} aNKSKLGANA ILGVSMAaar AaAAEKnVPL YqH1ADLsks KTSpYVLPVP
pileup.msf{Eno_clado} tNKtKiGANA ILGVSMAVaK AaAAEKrVPL YaHIsDLsGt K.kpFVLPVP
Consensus -NK-K-GANA ILGVS-A--- A-AA----PL Y-H---L--- ------LPVP

151 200
pileup.msf{enog_human} AFNVINGGSH AGNKLAMQEF MILPvGAESF RDAMRLGAEV YHTLKgVIKd
pileup.msf{enog_rat} AFNVINGGSH AGNKLAMQEF MILPvGAESF RDAMRLGAEV YHTLKgVIKd
pileup.msf{enog_mouse}, AFNVINGGSH AGNKLAMQEF MILPvGAESF RDAMRLGAEV YHTLKgVIKd
pileup.msf{eno_drome} AFNVINGGSH AGNKLAMQEF MILPtGAtSF tEAMkMGsEV YHhLKnVIKa
pileup.msf{eno1_yeast} fLNV1NGGSH AGgaLALQEF MIaPtGAktF aEALRiGsEV YHnLKsltKk
pileup.msf{eno2_yeast} fLNV1NGGSH AGgaLALQEF MIaPtGAktF aEAMRiGsEV YHnLKsltKk
pileup.msf{Eno_clado} fMNVVNGGSH AGgrLAFQEF MIvPsGApSF tEAMRqGAEV YqkLKsltKk
Consensus --NV-NGGSH AG--LA-QEF MI-P-GA--F --A---G-EV Y--LK---K-

201 250
pileup.msf{enog_human} KYGKDATNVG DEGGFAPNI1 eNsEALELVK EAIDKAGYTe KmvIGMDVAA
pileup.msf{enog_rat} KYGKDATNVG DEGGFAPNI1 eNsEALELVK EAIDKAGYTe KmvIGMDVAA
pileup.msf{enog_mouse} KYGKDATNVG DEGGFAPNI1 eNsEALELVK EAIDKAGYTe KmvIGMDVAA
pileup.msf{eno_drome} KFG1DATaVG DEGGFAPNIQ sNkEALnLIs DAIaKAGYTG KIeIGMDVAA
pileup.msf{eno1_yeast} rYGasAgNVG DEGGvAPNIQ taeEALDLIv DAIkaAGhdG KVkIGLDcAs
pileup.msf{eno2_yeast} rYGasAgNVG DEGGvAPNIQ taeEALDLIv DAIkaAGhdG KVkIGLDcAs
pileup.msf{Eno_clado} rYGqsAgNVG DEGGvAPdIQ taeEALDLIt DAIEeAGYTG qIkIaMDVAs
Consensus --G--A--VG DEGG-AP-I- ---EAL-L-- -AI--AG--- ---I--D-A-

251 300
pileup.msf{enog_human} SEFYrDG..K YDLDFKs.Pt DpSrYITGDQ LgaLYQDFVr dYPVVSIEDP
pileup.msf{enog_rat} SEFYrDG..K YDLDFKs.Pa DpSrCITGDQ LgaLYQDFVr nYPVVSIEDP
pileup.msf{enog_mouse} SEFYrDG..K YDLDFKs.Pa DpSrYITGDQ LgaLYQDFVr nYPVVSIEDP
pileup.msf{eno_drome} SEFYKDG..q YDLDFKNekS DKSqW1paDk LAnLYkEFIk dFPIVSIEDP
pileup.msf{eno1_yeast} SEFFKDG..K YDLDFKNPnS DKSkW1TGpQ LAdLYhsLmk rYPIVSIEDP
pileup.msf{eno2_yeast} SEFFKDG..K YDLDFKNPeS DKSkW1TGve LAdMYhsLmk rYPIVSIEDP
pileup.msf{Eno_clado} SEFYKadekK YDLDFKNPdS DKSkWITyEQ LAdqYnELaa kYPIVSIEDP
Consensus SEF------- YDLDFK---- D-S------- L---Y----- --P-VSIEDP

301 350
pileup.msf{enog_human} FDQDDWaAWS KF..TAnVGI QIVGDDLTVT NPKRIerAVE eKACNCLLLK
pileup.msf{enog_rat} FDQDDWaAWS KF..TAnVGI QIVGDDLTVT NPKRIerAVE eKACNCLLLK
pileup.msf{enog_mouse} FDQDDWaAWS KF..TAnVGI QIVGDDLTVT NPKRIerAVE eKACNCLLLK
pileup.msf{eno_drome} FDQDhWEAWS nL..TgctdI QIVGDDLTVT NPKRIatAVE kKACNCLLLK
pileup.msf{eno1_yeast} FaeDDWEAWS hFFKTAg..I QIVaDDLTVT NPKRIatAIE kKAadaLLLK
pileup.msf{eno2_yeast} FaeDDWEAWS hFFKTAg..I QIVaDDLTVT NPaRIatAIE kKAadaLLLK
pileup.msf{Eno_clado} FaeDDWEAWS yFYKTsgsdf QIVGDDLTVT NPefIkxAIE tKACNaLLLK
Consensus F--D-W-AWS ----T----- QIV-DDLTVT NP--I--A-E -KA---LLLK

351 400
pileup.msf{enog_human} VNQIGsVTEA IQAcKLAqeN GWGVMVSHRS GETEDTFIAD LVVGLcTGQI
pileup.msf{enog_rat} VNQIGsVTEA IQAcKLAqeN GWGVMVSHRS GETEDTFIAD LVVGLcTGQI
pileup.msf{enog_mouse} VNQIGsVTEA IQAcKLAqeN GWGVMVSHRS GETEDTFIAD LVVGLcTGQI
pileup.msf{eno_drome} VNQIGTVTEs IaAhlLAkkN GWGtMVSHRS GETEDsFIgD LVVGLsTGQI
pileup.msf{eno1_yeast} VNQIGT1sEs IkAaqdsfaa GWGVMVSHRS GETEDTFIAD LVVGLrTGQI
pileup.msf{eno2_yeast} VNQIGT1sEs IkAaqdsfaa nWGVMVSHRS GETEDTFIAD LVVGLrTGQI
pileup.msf{Eno_clado} VNQIGTITEA InAaKdsfaa GWGVMVSHRS GETEDvtIAD iVVGLraGQI
Consensus VNQIG---E- I-A------- -WG-MVSHRS GETED--I-D -VVGL--GQI

401 442
pileup.msf{enog_human} KTGAPCRSER LAKYNQLMRI EEELGDEARF AGhNFRNPSV L.
pileup.msf{enog_rat} KTGAPCRSER LAKYNQLMRI EEELGEEARF AGhNFRNPSV L.
pileup.msf{enog_mouse} KTGAPCRSER LAKYNQLMRI EEELGDEARF AGhNFRNPSV L.
pileup.msf{eno_drome} KTGAPCRSER LAKYNQiLRI EEEiGagvkF AGksFgkPq. ..
pileup.msf{eno1_yeast} KTGAPaRSER LAK1NQLLRI EEELGDnAvF AGeNFhhgdk L.
pileup.msf{eno2_yeast} KTGAPaRSER LAK1NQLLRI EEELGDkAvY AGeNFhhgdk L.
pileup.msf{Eno_clado} KTGAPaRSER LAK1NQiLRI EEELGDkr1Y AGdNFRtain Lz
Consensus KTGAP-RSER LAK-NQ--RI EEE-G----- AG--F----- --

Es kann in diesem Fall die Verbindung zwischen Proteinfunktion und Allergenität nicht hergestellt werden. Enolasen sind aber aus einem anderen Punkt heraus interessant. Bei Saccharomyces cerevisiae konnte die Enolase (ENO1) als "heat-shock-Protein" nachgewiesen werden. Die Expression von ENO1 wird hier als Mehrgebrauch von Energie in dieser schwierigen Stressbedingung gedeutet (Iida und Yahara 1985). In Hefe wird Enolase auch in der stationären Phase sowie unter Schwefelhunger vermehrt exprimiert (Cohen 1987).

Die nachfolgende Sequenz 3 zeigt mit Computerunterstützung gefundene B-Zellepitope. Hoher Antigenindex, unter Berücksichtigung von Sekundärstruktur, Oberflächenlage, Hydrophilität, Flexibilität etc.

### Sequenz 3: Clah47=Enolase_clado: B-Zellepitope

(1) ANGABEN ZU SEQ ID NO:3
   (i) SEQUENZKENNZEICHEN:
      (A) LÄNGE: einzeln angeführt
      (B) ART: Protein
   (ii) ART DES MOLEKÜLS: Peptide
   (iii) HYPOTHETISCH: nein
   (v) ART DES FRAGMENTS: N-Terminus bis C-Terminus
   (vi) URSPRÜNGLICHE HERKUNFT:
      (A) ORGANISMUS: Cladosporium herbarum
      (C) ENTWICKLUNGSSTADIUM: Sporen und vegetative Hyphen

Tyr Val Tyr Asp Ser Leu Gly Asn Pro Thr Val Glu Val (10-22)

Pro Ser Val Ala Ser Thr Gly Ser His Glu Ala Cys Glu Leu Arg Asp Gly Asp Lys Ser Lys Trp

Ala Gly Lys Gly Val Thr Lys (36-64)

Asn Lys Leu Asp Gly Thr Thr Asn Lys Thr Lys Ile Gly Ala (95-108)

Ala His Ile Ser Asp Leu Ser Gly Thr Lys Lys Pro Phe Val (132-145)

Tyr Gln Lys Leu Lys Ser Leu Thr Lys Lys Arg Tyr Gly Gln Ser Ala Gly Asn Val Gly Asp Glu

(190-211)

Phe Lys Asn Pro Asp Ser Asp Lys Ser Lys Trp Ile Thr Tyr Glu (264-278)

Ser Tyr Phe Tyr Lys Thr Ser Gly Ser Asp Phe (309-319)

Ala Ile Asn Ala Ala Lys Asp Ser Phe Ser Ala Gly Trp Gly (359-372)

Met Val Ser His Arg Ser Gly Glu Thr Glu Asp Val Thr Met (374-387)

His Ile Val Arg Arg Ser Arg Ala Gly Gln Ile Lys Thr Gly  (388-401)

Ala Pro Ala Arg Ser Asp Gly Leu Ala Lys Leu Asn  (402-413)

Ile Leu Arg Ile Glu Glu Glu Leu Gly Asp Lys Arg Leu Tyr Ala Gly Asp Asn Phe Arg Thr Ala

 (415-436)

Die nachfolgende Sequenz 4 zeigt die berechneten T-Zellepitope im 1-Lettercode. Amphipathische Bereiche mit einem Score geringer als 10 wurden für nicht relevant angenommen.

### Sequenz 4: Vorausgesagte amphipathatische Segment

T-Zellepitope
------------------
IHSRYVYDSLGN
KGVTKAVANVNEIIAP
DAFLNKLDGT
AHISDLSG
PSFTEAMRQGAEVYQKLKSLTK
GQSAGNV
EALDLITDAIEE
LADQYNEL
EAWSYFY
VNQIGTITEAINAAK
DGLAKLNQILRIEE

(1) ANGABEN ZU SEQ ID NO:4
   (i) SEQUENZKENNZEICHEN:
      (A) LÄNGE: einzeln angeführt
      (B) ART: Protein
   (ii) ART DES MOLEKÜLS: Peptide
   (iii) HYPOTHETISCH: nein
   (v) ART DES FRAGMENTS: N-Terminus bis C-Terminus
   (vi) URSPRÜNGLICHE HERKUNFT:
      (A) ORGANISMUS: Cladosporium herbarum
      (C) ENTWICKLUNGSSTADIUM: Sporen und vegetative Hyphen

Ile His Ser Arg Tyr Val Tyr Asp Ser Leu Gly Asn (6-17)

Lys Gly Val Thr Lys Ala Val Ala Asn Val Asn Glu Ile Ile Ala Pro (60-75)

Asp Ala Phe Leu Asn Lys Leu Asp Gly Thr (91-100)

Ala His Ile Ser Asp Leu Ser Gly (132-139)

Pro Ser Phe Thr Glu Ala Met Arg Gln Gly Ala Glu Val Tyr Gln Lys Leu Lys Ser Leu Thr Lys

(177-198)

Gly Gln Ser Ala Gly Asn Val (202-208)

Glu Ala Leu Asp Leu Ile Thr Asp Ala Ile Glu Glu (223-234)

Leu Ala Asp Gln Tyr Asn Glu Leu (280-287)

Glu Ala Trp Ser Tyr Phe Tyr (306-312)

Val Asn Gln Ile Gly Thr Ile Thr Glu Ala Ile Asn Ala Ala Lys (350-364)

Asp Gly Leu Ala Lys Leu Asn Gln Ile Leu Arg Ile Glu Glu (407-420)

Die T-Zellepitope errechnen sich aus den Aminosäurepositionen der Midpoints, die N-terminal von einem Lysin (K), C-terminal von einem Prolin (P) flankiert werden (=Flags). Es sind nur dann potentielle T-Zellepitope vorhanden, wenn der "Score-Index" größer als 10 ist.

### Literatur

Aukrust, L. (1979).
   Cross radioimmunoelectrophoretic studies of distinct allergens in two extracts of Cladosporium herbarum.
   Int. Arch. Allergy Appl. Immunol. 58, 371.
Aukrust, L. (1980).
   Allergens in Cladosporium herbarum.
   In Advances in Allergology and Immunology. Edited by A. Oehling. Oxford, Pergamon Press.
Birkner, T., Rumpold, H., Jarolim, E. Ebner, H., Breitenbach, M., Skarvil, F., Scheiner, O., Kraft, D. (1990).
   Evaluation of immunotherapy-induces changes in specific IgE, IgG and IgG subclasses in birch pollen allergic patients by means of immunoblotting. Correlation with clinical response.
   Allergy 45, 418.
Bold, H.C., Alexopoulos, C.J., Delevoryas, T. (1973).
   Morphology of plants and fungi.
   New York, Harper and Row.
Cohen, R.T., Yokoi, J.P., Holland, A.E., Pepper, A.E., Holland, M.J. (1987).
   Transcription of the constitutively expressed yeast enolase gene ENO1 is mediated by positive and negative cis-acting regulatory sequences.
   Mol. Cell. Biol. 7, 2753.
Ferreira, F.D., Hoffmann-Sommergruber, K., Breiteneder, H., Pettenburger, K., Ebner, C., Sommergruber, W., Steiner, R., Bohle, B., Sperr, W.R., Valent, P., Kungl, A.J., Breitenbach, M., Kraft, D., Scheiner, O. (1993).
   Purification and characterization of recombinant BetvI, the major birch pollen allergen. Immunological equivalence to natural BetVI.
   J. Biol. Chem. in press.
Foucard, T. Dreborg, S., Sten, E. (1984).
   Mould Allergy Workshop. Uppsala, Sweden:
   Ord & Form; Pharmacia Diagnostics AB 1984.
Francoeur, A.M., Peebles, C.L., Heckman, K.J., Lee, J.C., Tan, E.M. (1985).
   Identification of ribosomal protein autoantigens.
   J. Immunol. 135, 1767.
Gell, P.G.H., Coombs, R.R.A., Lachmann, P.J. (1975).
   Clinical Acpects of Immunology.
   Blackwell, Oxford.
Gravesen, S. (1979).
   Fungi as a cause of allergic disease.
   Allergy 34, 135.
Harada, S., Agarwal, D.P., Goedde, H.W. (1982).
   Mechanism of alcohol sensitivity and disulfiram-ethanol reaction.
   Subst. Alco. Act. Misuse. 3, 107.
Hines, J.J., Weissbach, H., Brot, N., Elkon, K. (1991).
   Anti-P autoantibody production requires P1/P2 as immunogens but is not driven by exogenous self-antigen in mrl mice.
   J. Immunol., 146, 3386.
Hsu, L.C., Bendel, R.E., Yoshida, A. (1987).
   Direct detection of usual and atypical alleles on the human aldehyde dehydrogenase-2 (ALDH2) locus.
   Am. J. Hum. Genet. 41, 996.
Iida, H., Yahara, i. (1985).
   Yeast heat shock protein of MW 48000 is an isoprotein of enolase.
   Nature 315, 688.
Lacey, J. (1981).
   The aerobiology of conidial fungi.
   In Biology of conidial fungi. Vol 1.
   New York, Academic Press.
Margalit, H., Spogue, J.L., Cornette, J.L., Cease, K.B., Delisi, C., Berzofsky, J.A. (1987).
   Prediction of immunodominant Helper T cell antigenic sites from the primary sequence.
   J. Immunol. 138, 2213.
Rammensee, H.G., Falk, K., Rötzschke, O. (1993).
   MHC molecules as peptide receptors.
   Current Opinion in Immunol. 5, 35.
Rich, B.E., Steitz, J.A. (1987).
   Human acidic ribosomal phosphoproteins P0, P1 and P2: analysis of cDNA clones, in vitro synthesis and assembly.
   Mol. Cell. Biol. 7, 4065.
Rothbard, J.B., Gefter, M.L. (1991).
   Interactions between immunogenic peptides and MHC proteins.
   Ann. Rev. Immunol. 9, 527.
Sanger, F., Nicklen, S., Coulson, A.R. (1977).
   DNA sequencing with chain-terminating inhibitors.
   Proc. Natl. Acad Sci. USA 74, 5463-5468

## Patentansprüche

1. Rekombinantes DNA Molekül, das für Polypeptide das Allergen Clah47 kodiert, **dadurch gekennzeichnet, daß** es eine Nukleinsäuresequenz aufweist, die mit der Sequenz 1 übereinstimmt.

2. Rekombinantes DNA-Molekül nach Anspruch 1, **dadurch gekennzeichnet, daß** es Nukleinsäuresequenzen aufweist, die durch Degeneration aus der dargestellten Sequenzen 1 ableitbar ist.

3. Rekombinantes DNA-Molekül nach Anspruch 1 oder 2, **dadurch gekennzeichnet, daß** es funktionell mit einer Expressionskontrollsequenz zu einem Expressionskonstrukt verbunden ist.

4. Wirtssystem zur Expression von Polypeptiden, **dadurch gekennzeichnet, daß** es mit einem rekombinanten Expressionskonstrukt nach Anspruch 3 transformiert ist.

5. Rekombinantes oder synthetisches Protein oder ein Polypeptid nach Anspruch 4, **dadurch gekennzeichnet, daß** es eine Aminosäuresequenz aufweist, die der gezeigten Sequenz 1 entspricht.

6. Rekombinantes oder synthetisches Protein oder Polypeptid nach Anspruch 5, **dadurch gekennzeichnet, daß** es ein Fusionsprodukt darstellt, das die Antigenität des Allergens Clah47 aufweist und einen zusätzlichen Polypeptidanteil besitzt, wobei das gesamte Fusionsprodukt von der DNA eines Expressionskonstrukts gemäß Anspruch 3 kodiert wird.

7. Rekombinantes oder synthetisches Protein oder Polypeptid nach Anspruch 6, **dadurch gekennzeichnet, daß** der besagte zusätzliche Polypeptidanteil β-Galaktosidase oder ein anderes zur Fusion geeignetes Polypeptid ist.

8. Diagnostisches oder therapeutisches Reagens, **dadurch gekennzeichnet, daß** es ein synthetisches Protein oder Polypeptid gemäß einem der Ansprüche 5 bis 7 enthält.

9. Verfahren zum in vitro -Nachweis der Allergie eines Patienten gegen das Allergen Clah47, **dadurch gekennzeichnet, daß** die Reaktion der IgE Antikörper im Serum des Patienten mit einem rekombinanten oder synthetischen Protein oder Polypetid nach einem der Anprüche 5 bis 7 gemessen wird.

10. Verfahren zum in vitro - Nachweis der zellulären Reaktion auf das Allergen Clah47, **dadurch gekennzeichnet, daß** ein rekombinantes oder synthetisches Protein oder Polypeptid nach einem der Ansprüche 5 bis 7 zur Stimulierung oder Hemmung der zellulären Reaktion eingesetzt wird.

## Claims

1. Recombinant DNA molecule which codes for polypeptides Allergen Clah47, **characterised in that** it has a nucleic acid sequence which corresponds to sequence 1.

2. Recombinant DNA molecule as claimed in Claim 1, **characterised in that** it has nucleic acid sequences, which are derivable by degeneration from the shown sequences 1.

3. Recombinant DNA molecule as claimed in Claim 1 or 2, **characterised in that** it is functionally connected to an expression control sequence to form an expression construct.

4. Host system for expressing polypeptides, **characterised in that** it is transformed with a recombinant expression construct as claimed in Claim 3.

5. Recombinant or synthetic protein or a polypeptide as claimed in Claim 4, **characterised in that** it has an amino acid sequence which corresponds to the shown sequence 1.

6. Recombinant or synthetic protein or polypeptide as claimed in Claim 5 **characterised in that** it constitutes a fusion product, which has the antigenicity of the Allergen Clah47 and has an additional polypeptide component, whereby the entire fusion product is coded for by the DNA of an expression construct as claimed in Claim 3.

7. Recombinant or synthetic protein or polypeptide as claimed in Claim 6, **characterised in that** the said additional polypeptide component is β-galactosidase or another polypeptide suitable for fusion.

8. Diagnostic or therapeutic reagent, **characterised in that** it contains a synthetic protein or polypeptide as claimed in one of Claims 5 to 7.

9. Method of in vitro diagnosis of the allergy of a patient to the Allergen Clah47, **characterised in that** the reaction of the IgE antibodies in the serum of the patient is measured with a recombinant or synthetic protein or polypeptide as claimed in one of Claims 5 to 7.

10. Method of in vitro diagnosis of the cellular reaction to the Allergen Clah47, **characterised in that** a recombinant or synthetic protein or polypeptide as claimed in one of Claims 5 to 7 is used to stimulate or inhibit the cellular reaction.

## Revendications

1. Molécule d'ADN recombinant codant pour le polypeptide d'allergène Clah47, **caractérisée en ce qu'**elle présente la séquence nucléotidique conformément à la SEQ ID No.1.

2. Molécule d'ADN recombinant selon la revendication 1, **caractérisée en ce qu'**elle présente une séquence nucléotidique dégénérée par rapport à la SEQ ID No. 1.

3. Molécule d'ADN recombinant selon l'une des revendications 1 ou 2, **caractérisée en ce qu'**elle est liée fonctionnellement à une séquence de contrôle d'expression pour former un construit d'expression.

4. Système hôte pour l'expression de polypeptides, **caractérisé en ce qu'**il est transformé avec un construit d'expression recombinant selon la revendication 3.

5. Protéine recombinante ou synthétique ou polypeptide selon la revendication 4, **caractérisé(e) en ce qu'**elle ou il présente la séquence d'acides aminés correspondant à la SEQ ID No. 1.

6. Protéine recombinante ou synthétique ou polypeptide selon la revendication 5, **caractérisé(e) en ce qu'**elle ou il représente un produit de fusion qui présente l'antigénicité de l'allergène Clah47 et possède une séquence polypeptidique supplémentaire, le produit de fusion dans son intégralité étant codé par l'ADN d'un construit d'expression selon la revendication 3.

7. Protéine recombinante ou synthétique où polypeptide selon la revendication 6, **caractérisé(e) en ce que** ladite séquence polypeptidique supplémentaire est la β-galactosidase ou un autre polypeptide approprié à la fusion.

8. Réactif diagnostique ou thérapeutique, **caractérisé en ce qu'**il contient une protéine ou un polypeptide synthétique selon l'une des revendications 5 à 7.

9. Procédé de détection in vitro de l'allergie d'un patient à l'allergène Clah47, **caractérisé en ce que** l'on mesure la réaction des anticorps IgE du sérum du patient avec une protéine ou un polypeptide recombinant ou synthétique selon l'une des revendications 5 à 7.

10. Procédé de détection in vitro de la réaction cellulaire à l'allergène Clah47, **caractérisé en ce que** l'on met en oeuvre une protéine ou un polypeptide recombinant ou synthétique selon l'une des revendications 5 à 7 pour stimuler ou inhiber la réaction cellulaire.
